# EUROPÄISCHE PATENTSCHRIFT

(11) **EP 0 890 345 B1**
(45) Veröffentlichungstag und Bekanntmachung des Hinweises auf die Patenterteilung: **17.01.2007**
(21) Anmeldenummer: 98111380.6
(22) Anmeldetag: 20.06.1998
(51) Int. Cl.: A61C 8/00, A61B 17/80, A61F 2/28

(54) **Vorrichtung zur Regeneration vom menschlichen und tierischen Knochen, insbesondere im Zahnkieferbereich**
Device for regeneration of human or animal bone, in particular for the jawbone
Dispositif de régénération osseuse notamment pour la machoire

(30) Priorität: 09.07.1997 DE 19729222; 01.02.1998 DE 19803628
(43) Veröffentlichungstag der Anmeldung: 13.01.1999
(73) Patentinhaber: Essiger, Holger K., Dr., 30900 Wedemark (DE)
(72) Erfinder: Essiger, Holger K., Dr., 30900 Wedemark (DE)
(74) Vertreter: Leine, Sigurd

(56) Entgegenhaltungen:
- WO-A-91/14404
- WO-A-97/43978
- US-A- 4 484 570
- US-A- 5 246 369

## Beschreibung

Die Erfindung betrifft eine Vorrichtung zur Regeneration, Reparatur und Modellation von menschlichen und tierischen Knochen unter Einschluß jeglichen Knochenmaterials z. B. auf synthetischer Basis, insbesondere jedoch im zahnkieferbereich. Demgemäß ist die Vorrichtung auf allen Gebieten des menschlichen und tierischen Knochenbaus, der Reparatur und der Modellation verwendbar so auch nach komplizierten Frakturen mit Defekten, wobei die Vorrichtung auch nicht auf bestimmte Knochengestalten beschränkt ist.

Die Erfindung geht von der Erkenntnis aus, daß die für die Knochenregeneration verwendeten Materialien - z. B. autogener, zerkleinerter Knochen und/oder z. B. Bio-Oss (eingetragene Marke) - während der Einheilungs- und Aufbauphase vergleichsweise sehr druckempfindlich sind und dann atrophieren und resorbieren würden. Daher lassen sich bei den bekannten Regenerationsverfahren z. B. am menschlichen Kiefer Atrophien bzw. Einschnürungen und Schwunderscheinungen nicht vermeiden. Der erwünschte Effekt einer möglichst vollkommenen Regeneration insbesondere in vertikaler Dimension läßt sich somit praktisch nicht erzielen.

Die vorerwähnten und die weiterhin bekannten Stoffe, die der Regeneration und der Wiederherstellung des Knochens dienen - eingeschlossen sind hier auch Knochenblöcke -, werden nachstehend Materialien genannt

WO 9114404 offenbart eine Vorrichtung zur Regeneration von mit einer Aufbauschicht zu versehenden Knochen, bestehend aus: einem gebogenen, in vertikaler Richtung steifen Element, das den Bereich des erwünschten Knochenwachstums überbrückt, einem Implantat und einem Befestigungselement zur Befestigung des Elementes am Implantat, wobei es sich beim steifen Element um eine domartige Ausgestaltung handelt.

US 4,484,570 offenbart eine Vorrichtung zur Regeneration, Reparatur und Modellation von menschlichen und tierischen Knochen unter Einschluß jeglichen Knochenersatzmaterials, z. B. auf synthetischer Basis, insbesondere im Zahnkiefer-Bereich, mit einer biegsamen Schiene, um sich an die Kontur der miteinander zu verbindenden Knochenteile anzupassen.

Nachteilig hierbei ist die Tatsache, daß eine Metallplatte, die auf die miteinander zu verbindenden Knochenteile aufgelegt wird, mittels Schrauben gegen die entsprechenden Knochenteile gespannt wird, so daß eine erhebliche Druckkraft auf die Knochenteile ausgeübt wird, was zu einer Atrophierung führt.

Dementsprechend liegt der Erfindung im wesentlichen die Aufgabe zugrunde, die erwähnten Nachteile der Atrophie zu vermeiden, also Einschnürungen zwischen Implantaten und allen anderen Knochenanlagerungs- und Reparaturstellen sowie dementsprechende Konturveränderungen weitgehend zu vermeiden. (1)

Diese Aufgabe wird erfindungsgemäβ gelöst durch die Vorrichtung nach Anspruch 1. Bevorzugte Ansführungsformen der Erfindung sind Gegenstand der Unteransprüche.

Diese Schiene nimmt Druckkräfte auf und verhindert somit weitgehend eine Druckbeanspruchung der Materialien. Weiterhin werden durch die Schiene örtlich einwirkende Kräfte auf größere Bereiche verteilt, um so die spezifische Pressung zu verringern. Zugleich werden durch die Schiene aber auch die zugehörigen Implantate geschützt und stabilisiert. Außerdem bietet die Schiene noch den Vorteil, die bei Regenerationsverfahren benutzten Membranen, die insbsondere zum Fernhalten des Bindegewebes dienen, zu haltern und ggf. zu befestigen, indem die Membranen über die Scheine geführt und von dieser unterfangen werden.

Zweckmäßigerweise erhält die Schiene zwei oder mehrere, über ihre Länge verteilte Durchbrechungen, vorzugsweise Langlöcher, durch die Befestigungselemente z. B. Stifte oder Schrauben geführt sind, welche im Implantat gehaltert z. B. eingeschraubt sind. Die Langlöcher haben dabei den Vorteil, daß die Schiene an Implantaten mit ungleichen gegenseitigen Abständen gehaltert werden kann.

Während bekannte Implantate für den Zahnkieferbereich außen mit Aufrauhungen, Vertiefungen oder Durchbrechungen versehen sind, werden aufgrund der Erfindung stiftförmige z. B. zylinderförmige Implantate vorgeschlagen, die außen poliert bzw. derart glatt ausgeführt sind, daß sie im eingesetzten Zustand mit Kraftaufwand um ihre Längsachse gedreht werden können, und zwar auch nach schon längerer Knochenverweildauer. Besonders vorteilhaft ist es hierfür, wenn die Implantate mit einem Außengewinde versehen sind. Diese Implantate sind durch Verdrehen höhenverstellbar und sind im Falle eines in Stufen zu vollziehenden Knochenaufbaues in ihrer Höhenlage veränderbar z. B. um etwa je 4 mm in mehreren Schüben nach Beginn einer z. B. circa neunmonatigen Aufbauphase.

Nach der Regeneration können die Implantate 4 entfernt werden oder Tragelemente für Zahnelemente oder dergleichen oder aber auch für andere Gebrauchselemente bilden.

Weitere Einzelheiten der Erfindung werden anhand der Zeichnung erläutert, in der Ausführungsbeispiele der Erfindung dargestellt sind. Es zeigen :
- Fig. 1: eine Draufsicht auf einen Längenabschnitt einer für die Regeneration von Kieferknochen verwendbaren Schiene,
- Fig. 2: den Schienenabschnitt gemäß Fig. 1 in der Seitenansicht,
- Fig. 3: einen gegenüber Fig. 1 abgewandelten Schienenabschnitt in der Draufsicht,
- Fig. 4: den Schienenabschnitt gemäß Fig. 3 in der Seitenansicht,
- Fig. 5: ein in Verbindung mit Fig. 1 - 4 verwendbares Implantat in der Ansicht,
- Fig. 6: einen Schnitt entlang der Linie VI - VI von Fig. 3 zusammen mit einem Implantat gemäß Fig. 5,
- Fig. 7: einen schematisch wiedergegebenen Längsschnitt durch einen zu regenerierenden Kieferknochen im Regenerationsbereich und
- Fig. 8: einen Teilschnitt durch ein Befestigungselement für eine Schiene.

Die aus z. B. Titan oder einem anderen geeigneten Material gefertigte Schiene 1 wird als im Querschnitt flaches Gebilde größerer Längenerstreckung hergestellt und hat - im Querschnitt gesehen - mittig Durchbrechungen in Form von runden Löchern, vorzugsweise aber über die Schienenlänge verteilt angeordnete Langlöcher 3, die eine bessere Anpassung an nicht präzise plazierbare Implantate 4 zulassen. Die Schiene 1 weist an beiden Rändern Einkerbungen 5 auf und zudem an der Unterseite Quernuten 6. Diese Querschnittsverjüngungen 5, 6 ermöglichen ein Ablängen und Verformen einer in längeren Abschnitten hergestellten Schiene 1 zu einem gewünschten Schienenabschnitt. Die Quernuten 6 gestatten dabei ein vereinfachtes Ablängen, während die Einkerbungen 5 eine Biegeverformung zur Anpassung an z. B. die Kieferkrümmung des Patienten erleichtern sollen (Sollbiegestellen).

Die stiftförmigen Implantate 4 bestehen ebenfalls aus z. B. Titan oder einem anderen geeigneten Material und weisen im gesamten zentralen Schaftbereich ein Außengewinde 7 auf und sind im übrigen außen äußerst glatt bzw. poliert ausgeführt, um das Implantat 4 in der Wirkstellung, also im implantierten Zustand um seine Längsachse verdrehen bzw. mehr oder weniger herausschrauben ggf. aber auch hereinschrauben zu können. Dazu kann das Implantat 4 am oberen Ende besonders gestaltet sein z. B. mit einem Innensechskant ausgestattet werden, um eine Verdrehbewegung zu erleichtern. Das Implantat 4 hat zudem eine Längsbohrung 8 als Sackloch vom oberen Ende her, das zur Halterung eines mit einem Linsenkopf 9 versehenen Befestigungselementes 10 z. B. nach Art einer Schraube ausgeführt sein kann, aber auch ein am Kopf verbreiterter Stift sein kann. Das Befestigungselement 10 kann ggf. aber auch nach Entfernen der Schiene 1 im Bereich des Implantats 4 eine Überdeckung für das Implantat 4 bzw. dort ein Veschlußelement bilden.

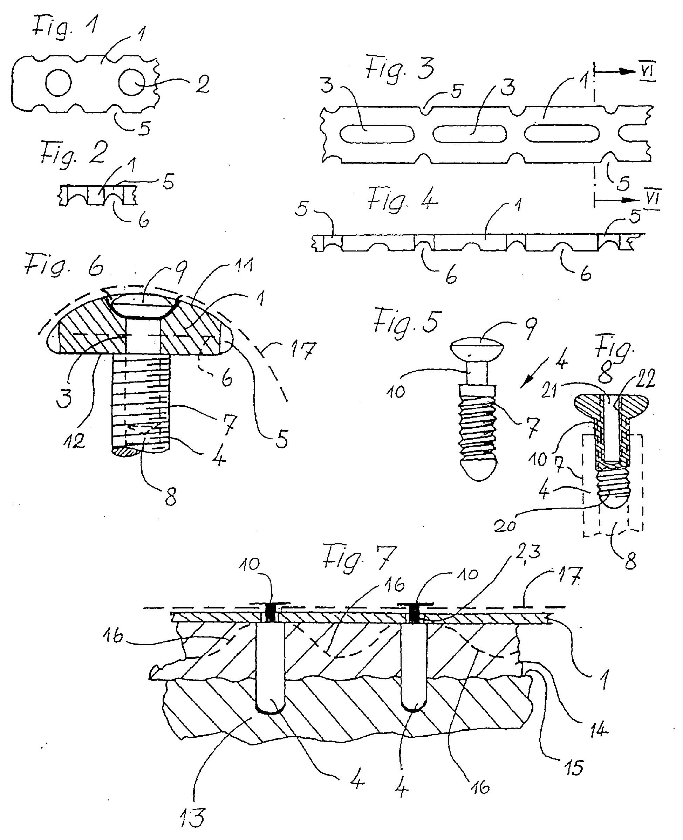

Der Durchmesser der Löcher 2 bzw. die Breite der Langlöcher 3 ist dem Durchmesser des Befestigungselementes 10 angepaßt, während der Durchmesser des Implantates 7 geringfügig größer ist als der vorerwähnte Durchmeser bzw. die vorgenannte Breite. Damit kann die abgelängte Schiene 1 gemäß Fig. 6 auf die Implantate 7 aufgesetzt und dort mit Hilfe der Befestigungselemente 10 befestigt werden. Da die Löcher 2 bzw. Langlöcher 3 sich an der Oberseite der Schiene 1 erweitern, kann der Linsenkopf 9 gemäß Fig. 6 versenkt werden, um so einen Überstand zu unterbinden.

Wie aus Fig. 6 ferner erkennbar ist, ist die Oberseite 11 der Schiene gewölbt bzw. konvex gestaltet, um im Wirkzustand Perforationen des Weichgewebes bzw. des ggf. dort befindlichen synthetischen Materials zu unterbinden. Die Unterseite 12 der Schiene 1 ist im wesentlichen eben gestaltet.

In Fig. 7 ist der zu regenerierende bzw. aufzubauende Knochen mit 13 und die durch die Materialien gebildete Aufbauschicht mit 14 bezeichnet. Die Ausgangskontur ist mit 15 gekennzeichnet.

Unterstellt man zunächst die Anordnung von mehreren Implantaten 4 und das Vorhandensein der Aufbauschicht 14 - jedoch ohne Schiene 1 und die besondere Ausbildung der Implatate 4 gemäß Fig. 5 - so ergibt sich bei Anwendung konventioneller Methoden nach freier Knochenkammverpflanzung infolge der eintretenden Atrophie ein Konturenverlauf der gestrichtelten Linie 16 entsprechend. Wird hingegen aufgrund der Erfindung eine Schiene 1 vorgesehen, so entfällt die Druckbelastung der Aufbauschicht 14 unter Aufrechterhaltung ihrer Aufbauschichthöhe. Die Schiene 1 überbrückt aber nicht nur die zwischen den Implantaten 4 befindlichen Regenerationsstellen, sie stabilisiert ihrerseits auch die Implantate 4.

Das Vorhandensein der Schiene eröffnet aber auch noch eine besondere Anordnung einer Membran 17 insbesondere zum Schutz der Aufbauschicht 14 bzw. zum Separieren und Abhalten des Bindegewebes. Die Membran 17 überdeckt auch die Schiene 1 und kann dort auch durch die Linsenköpfe 9 fixiert werden, wobei die Befestigungselemente 10 die Membran 17 durchsetzen und zwischen Schiene 1 und Linsenkopf 9 einklemmen können.

Nach Abschluß der gewünschten Regeneration und/oder Appositon werden die Membran 17 und die Schiene 1 entfernt.

Es sei noch erwähnt, daß - wie aus Fig. 3 hervorgeht - die Einkerbungen 5 nicht den Langlöchern 3 benachbart anzuordnen sind, um so bei der Verformung der gestreckt hergestellten Schiene 1 in die im wesentlichen u-förmige Kiefergestalt bzw. einem gekrümmten Verlauf eines anderen Knochens entsprechend eine Breitenverminderttng der Langlöcher 3 weitgehend auszuschalten.

Ferner sei hervorgehoben, daß die dargestellte mechanische Verbindung zwischen der Schiene 1 und dem Implantat 4 zwar vorteilhaft ist, daß sie ggf. aber durch andere Verbindungsarten ersetzt werden kann. Wichtig ist aber in jedem Falle, daß die eingesetzte Schiene 1 im Bereich ihrer Oberseite 11 ballig bzw. abgerundet geformt ist und Perforationen der Schleimhaut oder anderer Gewebe oder Materialien und somit auch der Membran 17 verhindert werden.

Schließlich sei auch darauf verwiesen, daß die Erfindung auch solche Schienen 1 umfassen soll, die schon bei ihrer Herstellung die dem zu behandelnden Knochen typische Gestalt z. B. bei Kiefern eine ungefähre U-Form (im Grundriß gesehen) bekommen haben.

Wenngleich auch die aufgrund der Erfindung vorgesehene Schiene mit ihren Stützstellen vorzugsweise bei der Knochenregeneration des menschlichen Kiefers Anwendung finden soll, kann sie sinngemäß auch an anderen Stellen des Knochengerüstes für Mensch und Tier z. B. auch bei Frakturen angewendet werden.

Nach der Behandlung wird die Schiene 1 entfernt, jedoch liegt es auch im Rahmen der Erfindung, die Schiene 1 an der Operationsstelle zu belassen.

Die in Fig. 8 wiedergegebene Ausführungsform weist gegenüber Fig. 5 eine Besonderheit auf. In Fig. 8 ist zwar ebenfalls ein als Schraube ausgeführtes Befestigungselement 10 mit Außengewinde 20 für die Schiene 1 vorgesehen, jedoch weist hier das Befestigungselement 10 ein seinen Kopf und einen Teil seines Schaftes durchsetzendes, oben offenen Sackloch 21 mit Innengewinde 22 auf, das nach der Schienenmontage und der Anbringung feiner Durchbrechungen in evtl. vorhandenen Überdeckungen (Schleimhaut) zur Aufnahme von vorzugsweise schraubbaren Befestigungseinrichtungen für Prothesen, Brücken oder andere Kauhilfsmittel und/oder kosmetische Mittel und/oder außerhalb bzw. oberhalb der Haut liegende rekonstruktive Hilfsmittel dienen kann. Die Befestigung kann temporär, aber auch auf Dauer sein. Diese durch das Befestigungselement 10 erfüllte weitere Aufgabe hat den Vorteil, daß z. B. in der Kieferchirurgie dem meist zahnlosen Patienten das Kauen möglich wird. Zumindest sollen jedoch kosmetische Zwekke erfüllt werden können. Die nicht näher dargestellten Befestigungseinrichtungen z. B. konische Pfosten können beliebiger Art sein, auch ist nicht unbedingt ein Schraubgewinde erforderlich.

## Patentansprüche

1. Vorrichtung zur Regeneration, Reparatur und Modellation von menschlichen und tierischen, mit einer Aufbauschicht (14) zu versehenden Knochen (13) unter Einschluss jeglichen Knochenersatzmaterials z. B. auf synthetischer Basis, insbesondere im Zahnkieferbereich,
mit einer biegesteifen längsgestrackten Schiene (1), deren Unterseite (22) im vorsentlichen ober gestaltet ist zur Überbrückung der Aufbauschicht (14) von mindestens einer Regenerations-, Reparatur- oder Modellationsstelle am Knochen (13),
zumindest zwei im Abstand voneinander angeordneten Implantaten, die im Kopfbereich Befestigungselemente (10) für die längsgestreckte Schiene (1) aufweisen, wobei die längsgestreckte Schiene (1) auf den zumindest zwei im Abstand voneinander angeordneten Implantaten (4) abgestützt ist,
**dadurch gekennzeichnet, daß** der Durchmesser des Implantates (4) derart größer ist als der Durchmesser des Befestigungselementes (10), daß die längsgestreckte Schiene auf die Implantate (4) aufsetzbar ist und dort mit dem Befestigungselementen (10) festklemmbar ist.

2. Vorrichtung nach Anspruch 1, **dadurch gekennzeichnet, daß** die Schiene (1) mit zwei oder mehreren, über ihre Länge verteilt angeordneten Durchbrechungen (2,3) zur Anbringung der Schiene (1) an den insbesondere aus Titan bestehenden Implantaten (4) versehen ist.

3. Vorrichtung nach Anspruch 2, **dadurch gekennzeichnet, daß** die Durchbrechungen sich in Schienenlängsrichtung erstreckende Langlöcher (3) sind.

4. Vorrichtung nach Anspruch 1, **dadurch gekennzeichnet, daß** die Oberseite der Schiene (1) im wesentlichen konvex ausgeführt ist.

5. Vorrichtung nach Anspruch 1 oder 2, **dadurch gekennzeichnet, daß** die Schiene (1) durch Querschnittsverjüngungen gebildete Sollbiegestellen aufweist.

6. Vorrichtung nach Anspruch 5, **dadurch gekennzeichnet, daß** sich die Sollbiegestellen (5) an den seitlichen Rändern der Schiene befinden, und zwar vorzugsweise an den den Durchbrechungen (2,3) abgekehrten Stellen.

7. Vorrichtung nach Anspruch 5, **dadurch gekennzeichnet, daß** sich die Sollbiegestellen (6) an der Unterseite der Schiene befinden.

8. Vorrichtung nach Anspruch 5 - 7, **dadurch gekennzeichnet, daß** die Sollbiegestellen (6) Bruchstellen bilden und/oder die Schiene (1) Sollbruchstellen aufweist.

9. Vorrichtung nach Anspruch 1, **dadurch gekennzeichnet, daß** die Implantate (4) stiftförmig ausgebildet und außen z. B. unter Verzicht von Aufrauhungen, Ausnehmungen oder Durchbrechungen derart glatt ausgebildet sind, daß sie im Wirkzustand um ihre Längsachse verdrehbar sind.

10. Vorrichtung nach Anspruch 10, **dadurch gekennzeichnet, daß** die Implantate (4) mit einem Außengewinde (7) zur Höhenverstellung versehen sind.

11. Vorrichtung nach Anspruch 1, **dadurch gekennzeichnet, daß** die Befestigungselemente (10) in Ausnehmungen (8) der Implantate (4) einführbare nagel- oder schraubenartige Elemente sind.

12. Vorrichtung nach Anspruch 12, **dadurch gekennzeichnet, daß** die Köpfe (9) der Befestigungselemente in den oberen Querschnittsbereich der Schiene (1) versenkbar sind.

13. Vorrichtung nach Anspruch 1, **dadurch gekennzeichnet, daß** die als Schrauben oder Stifte ausgeführten Befestigungselemente (10) von oben zugängliche, ggf. mit Innengewinde versehene Längsbohrungen (21)aufweisen.

14. Vorrichtung nach Anspruch 13, **dadurch gekennzeichnet, daß** die Längsbohrungen (21) Sacklöcher sind.

## Claims

1. Device for the regeneration, repair and modellation of human and animal bones (13) to be provided with a buildup layer (14), with inclusion of any bone replacement material, for example on a synthetic basis, in particular in the jaw region, comprising a bending resistant, elongate rail (1), the lower side (12) of which is substantially flat, for bridging the buildup layer (14) of at least one regeneration, repair or modellation point on the bone (13), at least two implants arranged spaced apart from one another, which have fastening elements (10) in the head region for the elongate rail (1), the elongate rail (1) being supported on at least two implants (4) arranged spaced apart from one another, **characterised in that** the diameter of the implant (4) is greater than the diameter of the fastening element (10) in such a way that the elongate rail can be placed on the implants (4) and can be clamped there with the fastening elements (10).

2. Device according to claim 1, **characterised in that** the rail (1) is provided with two or more openings (2, 3) arranged distributed over its length for attaching the rail (1) to the implants (4) consisting, in particular, of titanium.

3. Device according to claim 2, **characterised in that** the openings are slots (3) extending in the longitudinal direction of the rail.

4. Device according to claim 1, **characterised in that** the upper side of the rail (1) is substantially convex.

5. Device according to claim 1 or 2, **characterised in that** the rail (1) has predetermined bending points formed by cross-sectional taperings.

6. Device according to claim 5, **characterised in that** the predetermined bending points (5) are located at the side edges of the rail and specifically preferably at the points remote from the openings (2, 3).

7. Device according to claim 5, **characterised in that** the predetermined bending points (6) are located on the underneath of the rail.

8. Device according to claim 5 to 7, **characterised in that** the predetermined bending points (6) form breaking points and/or the rail (1) has predetermined breaking points.

9. Device according to claim 1, **characterised in that** the implants (4) are pin-shaped and are smooth on the outside, for example dispensing with roughenings, recesses or openings, such that they can be rotated in the operating state about their longitudinal axis.

10. Device according to claim 9, **characterised in that** the implants (4) are provided with an external thread (7) for height adjustment.

11. Device according to claim 1, **characterised in that** the fastening elements (10) are nail-like or screw-like elements which can be introduced into recesses (8) of the implants (4).

12. Device according to claim 1, **characterised in that** the heads (9) of the fastening elements can be countersunk into the upper cross-sectional region of the rail (1).

13. Device according to claim 1, **characterised in that** the fastening elements (10) designed as screws or pins have longitudinal holes (21) which are accessible from above and optionally provided with an internal thread.

14. Device according to claim 13, **characterised in that** the longitudinal holes (21) are blind holes.

## Revendications

1. Dispositif de régénération, réparation et modelage d'os (13) humain ou animal destiné à être muni d'une couche structurale (14), moyennant l'insertion d'un matériau de remplacement de l'os, par exemple à base de matière synthétique, en particulier dans la région maxillaire, comportant un profilé allongé (1), résistant à la flexion, dont la face inférieure (12) est sensiblement plane et qui est destiné à couvrir la couche structurale (14) d'au moins une zone de régénération, réparation et modelage au niveau de l'os (13), au moins deux implants (4), qui sont situés à distance l'un de l'autre et qui, dans la partie supérieure, comportent des éléments de fixation (10) pour le profilé allongé (1), ledit profilé allongé (1) étant en appui sur lesdits au moins deux implants situés à distance l'un de l'autre, **caractérisé en ce que** le diamètre de l'implant (4) est supérieur au diamètre de l'élément de fixation (10) de telle sorte que le profilé allongé (1) peut être posé sur les implants (4) et y être fixé au moyen des éléments de fixation (10).

2. Dispositif selon la revendication 1, **caractérisé en ce que** le profilé (1) est muni de deux ou plusieurs trous traversants (2, 3), qui sont répartis sur la longueur dudit profilé et qui sont destinés au montage du profilé (1) contre les implants (4), réalisés en particulier en titane.

3. Dispositif selon la revendication 2, **caractérisé en ce que** les trous débouchants sont des trous oblongs (3) qui sont orientés dans le sens longitudinal du profilé.

4. Dispositif selon la revendication 1, **caractérisé en ce que** la face supérieure du profilé (1) est sensiblement convexe.

5. Dispositif selon la revendication 1 ou 2, **caractérisé en ce que** le profilé (1) comporte des points de flexion théorique formés par des rétrécissements de la section.

6. Dispositif selon la revendication 5, **caractérisé en ce que** les points de flexion théorique (5) sont disposés sur les bords latéraux du profilé, à savoir de préférence dans des zones éloignées des trous débouchants (2, 3).

7. Dispositif selon la revendication 5, **caractérisé en ce que** les points de flexion théorique (6) sont disposés sur la face inférieure du profilé.

8. Dispositif selon l'une quelconque des revendications 5 à 7, **caractérisé en ce que** les points de flexion théorique (6) forment des zones de rupture et/ou le profilé (1) comporte des zones de rupture théorique.

9. Dispositif selon la revendication 1, **caractérisé en ce que** les implants (4) sont réalisés en forme de pivot et sont lisses à l'extérieur, c'est-à-dire ne comportent ni rugosité, ni évidement, ni trou traversant, de telle sorte que, dans leur position active, ils sont aptes à tourner autour de leur axe longitudinal.

10. Dispositif selon la revendication 9, **caractérisé en ce que** les implants (4) sont munis d'un filetage (7) en vue de leur réglage en hauteur.

11. Dispositif selon la revendication 1, **caractérisé en ce que** les éléments de fixation (10) sont des éléments de type clou ou vis aptes à être introduits dans des évidements (8) des implants (4).

12. Dispositif selon la revendication 1, **caractérisé en ce que** les têtes (9) des éléments de fixation peuvent être noyées dans la zone transversale supérieure du profilé (1).

13. Dispositif selon la revendication 1, **caractérisé en ce que** les éléments de fixation (10), conçus sous forme de vis ou pivots, comportent des alésages longitudinaux (21) accessibles par le haut et, le cas échéant, taraudés.

14. Dispositif selon la revendication 13, **caractérisé en ce que** les alésages longitudinaux (21) sont des trous borgnes.
